# EUROPEAN PATENT SPECIFICATION

(11) **EP 3 530 126 B1**
(45) Date of publication and mention of the grant of the patent: **03.09.2025**
(21) Application number: 17863179.2
(22) Date of filing: 20.10.2017
(51) Int. Cl.: A23L 7/10, A23L 5/20, A23L 7/104, C12N 9/34

(54) **RICE MODIFIER**
REISMODIFIKATOR
MODIFICATEUR DE RIZ

(30) Priority: 21.10.2016 JP 2016207004
(43) Date of publication of application: 28.08.2019
(73) Proprietor: AJINOMOTO CO., INC., Chuo-ku Tokyo 104-8315 (JP)
(72) Inventor: OHIRA, Takuya, Kawasaki-shi Kanagawa 210-8681 (JP)
(74) Representative: Strehl & Partner mbB
(86) International application number: PCT/JP2017/037974
(87) International publication number: WO 2018/074582

(56) References cited:
- WO-A1-2014/001351
- WO-A1-2016/138315
- JP-A- 2008 161 144
- JP-A- 2009 022 267
- JP-A- 2011 193 876
- JP-A- 2013 240 322
- JP-A- 2016 154 489
- JP-A- S60 164 446
- S. CHIBA: "Molecular mechanism in a-glucosidase and glucoamylase", BIOSCIENCE, BIOTECHNOLOGY, BIOCHEMISTRY, vol. 61, no. 8, 1 January 1997 (1997-01-01), pages 1233 - 1239, XP055664704
- KUMAR PARDEEP ET AL: "Microbial glucoamylases: characteristics and applications", CRC CRITICAL REVIEWS IN BIOTECHNOLOGY, CRC PRESS, BOCA RATON, FL, US, vol. 29, no. 3, 1 January 2009 (2009-01-01), pages 225 - 255, XP009173351, ISSN: 0738-8551, DOI: 10.1080/07388550903136076
- TERASHIMA TERUYA: "Investigating sake brewing characteristics of toyama prefecture grown "Oyama-niski" rice for sake brewing", FOOD TRIALS AND RESEARCH, vol. 38, 2003, pages 68 - 70, XP009515119
- IMAI YASUHIKO: "Streamlining Japanese sake brewing management kit", FOOD INDUSTRY, vol. 39, no. 5, 15 March 1996 (1996-03-15), pages 68 - 74, XP009514266, ISSN: 0559-8990

## Description

### [Technical Field]

The present invention relates to a grain food modifying agent containing particular enzymes, a grain food containing the modifying agent and a production method thereof.

### [Background Art]

It is known that the texture (elasticity, stickiness, etc.) of cooked rice deteriorates over time and becomes hard and crumbly. Such phenomenon in which the moisture is separated and the gelatinized starch becomes hard after standing is generally called aging. This aging phenomenon becomes remarkable particularly when stored at low temperatures, thus causing a problem in long-term storage and distribution over long distance. For this reason, cooked rice that does not become hard even when stored and distributed in cold storage is desired.

On the other hand, cooked rice such as lunch box and the like preserved in cold storage is often heated with microwave oven and the like before eating. Moisture evaporates from the cooked rice after heating and the cooked rice shows a dry texture. Thus, cooked rice with good texture and water retention is desired.

To obtain such cooked rice, it has been reported to cook rice by adding various enzymes such as amylase (patent documents 1, 2), glucoamylase (patent document 3), α-glucosidase (patent documents 4 - 7) and the like. However, production suitability poses a problem since cooking rice by adding enzymes such as amylase and the like causes burning and sticking of the cooked rice to a rice cooker pot (patent document 8). Furthermore, it is also desired to simultaneously solve problems other than the improvement of the texture of cooked rice, such as difficult loosening during stirring due to excessive viscosity peculiar to starch, formability when producing rice ball and the like, and the like.

WO 2014/001351 A1 discloses a process of contacting rice with enzymes such as glucoamylase and α-glucosidase to provide cooked rice having retarded staling and improved sensory and texture properties.

### [Document List]

### [Patent documents]

patent document 1: JP-A-2015-525564
patent document 2: WO15/060374
patent document 3: JP-A-2016-154489
patent document 4: WO14/115894
patent document 5: JP-A-2011-206048
patent document 6: JP-A-2011-193876
patent document 7: JP-A-2010-35858
patent document 8: JP-A-2004-290075

### [SUMMARY OF THE INVENTION]

### [Problems to be Solved by the Invention]

The present invention aims to provide cooked rice showing suppressed aging and enhanced water retention, and provide cooked rice free of burning and sticking during cooking rice, easily loosened and superior in formability.

### [Means of Solving the Problems]

The present inventors have conducted intensive studies in an attempt to solve the aforementioned problems and found that cooked rice containing glucoamylase and α-glucosidase has good texture, suppresses aging even during low temperature preservation and maintains moisture even after heating, and further found that it is superior in the suppression of sticking to the rice cooker pot during cooking rice and in formability when forming same after rice cooking, which resulted in the completion of the present invention.

The present invention is defined in the appended claims.

### [Effect of the Invention]

According to the present invention, springy cooked rice superior in preservation property can be provided.

According to the present invention, cooked rice giving no dry feeling even after heating can be provided.

According to the present invention, sticking to a rice cooker pot during cooking does not occur and a loss of the starting material can be suppressed.

According to the present invention, cooked rice that is easily loosened during mixing and superior in formability can be provided.

### [Description of Embodiments]

The present invention relates to a grain food modifying agent containing glucoamylase and α-glucosidase (hereinafter sometimes to be also abbreviated as the modifying agent of the present invention).

As glucoamylase (hereinafter sometimes to be also abbreviated as GA) to be used in the present invention, a commercially available product or one prepared from a culture medium of a microorganism that produces GA can be used. As the preparation method thereof, a known protein separation and purification method (centrifugation, UF concentration, salting out, various chromatography using ion exchange resin and the like, etc.) can be used.

The kind of GA is not particularly limited as long as it has an action to cleave α-1,4-glucoside bond of amylose and amylopectin as constituent elements of starch from the non-reducing terminal with the glucose unit and/or an action to cleave α-1,6-glucoside bond. Examples of such enzyme include, but are not particularly limited to, GA and the like derived from genus Aspergillus, genus Rhizopus.

As α-glucosidase (hereinafter sometimes to be also abbreviated as AG) to be used in the present invention, a commercially available product or one prepared from a culture medium of a microorganism that produces AG can be used. As the preparation method thereof, a known protein separation and purification method (centrifugation, ultrafiltration (UF) concentration, salting out, various chromatography using ion exchange resin and the like, etc.) can be used.

The kind of AG is not particularly limited as long as it has an action to hydrolyze non-reducing terminal α-1,4-glucoside bond to produce α-glucose. Examples of such enzyme include, but are not particularly limited to, AG and the like derived from genus Aspergillus and genus Trichoderma, and AG derived from genus Aspergillus is preferable.

The grain to be used in the present invention is a food material containing starch, for example, cereal grains such as rice, wheat, barley, Japanese millet, foxtail millet and the like, beans such as soybean, adzuki bean and the like, potatoes such as white potato, sweet potato, corn and the like. Among these, rice, wheat, barley and the like are preferable, and rice is more preferable. The grain may be purified or unpurified, and is preferably purified.

In the present invention, the grain food is not particularly limited as long as it is a food containing starch, and a food in which starch contributes to the texture and property of the food can be mentioned. Specifically, rice food (cooked rice (white rice, rice with mixed grains), vinegared rice (rice prepared for sushi), cooked rice with red beans, pilaf, fried rice, rice seasoned and cooked with various ingredients, white steamed glutinous rice, thin rice porridge, risotto, rice ball, sushi, lunch box and the like), rice processed product (Japanese confectionery, rice cake and the like), noodle (wheat noodle (Japanese wheat noodles, Chinese noodles, pasta and the like), soba, rice-powder noodle and the like), wheat processed food (gyoza sheet, deep fry batter and the like), processing food using potatoes such as white potato, sweet potato and other vegetables such as corn and the like as the starting material can be mentioned. Among these, rice food is particularly preferable. Frozen products, aseptic packaging products, retort products, dried products, canned foods thereof are also included.

The grain food modifying agent of the present invention improves quality such as texture and the like of grain foods or production suitability thereof. The texture refers to a sensation that occurs when a food is put in the mouth, such as chewiness, texture on the tongue and the like. In the case of rice, it refers to the elasticity, stickiness, water retention (dry feeling) and the like of cooked rice. The production suitability means whether or not it is suitable for production of the desired quality. In the case of rice, it refers to sticking to the bottom of a rice cooker pot during cooking rice and easiness of forming when cooked rice is formed after rice cooking and the like.

The modifying agent of present invention contains glucoamylase and α-glucosidase as active ingredients, the content of glucoamylase is 1.0x10⁻³ - 5.0x10⁻¹U, relative to 1U α-glucosidase. The modifying agent of the present invention may contain these enzymes together or in the form of a kit in which they are prepared separately and combined before use.

The modifying agent of the present invention may be added at any step in the production step of a grain food as mentioned below. For example, grain is washed, water (80 - 200 g) (water content ratio 80 - 200 wt%) is generally added per 100 g of the grain, the modifying agent of the present inventionis added and they are cooked.

The modifying agent of the present invention may further contain, besides the above-mentioned enzymes, excipients such as dextrin, starch, processed starch, reduced maltose and the like, seasoning such as meat extract and the like, proteins such as plant protein, gluten, white of egg, gelatin, casein and the like, protein hydrolysate, protein partially decomposed product, emulsifier, citrate, chelating agents such as polymerized phosphate and the like, reducing agents such as glutathione, cysteine and the like, other food additives such as alginic acid, lye water, fats and oils, dye, acidulant, flavor and the like.

The form of the modifying agent of the present invention may be a liquid, a paste, a granule or a powder, and may have any of these forms.

A grain food containing the modifying agent of the present invention is also encompassed in the present invention. As the grain food, those mentioned above can be mentioned.

The present invention also includes a production method of a grain food including a step of contacting glucoamylase and α-glucosidase with the grain (hereinafter sometimes to be also abbreviated as "the method of the present invention").

The method of the present invention is characterized in that it includes a step of contacting glucoamylase and α-glucosidase with the grain. As other steps, for example, the following conventionally steps may be applied:
(a) washing step of grain
(b) step of immersing grain in water
(c) step of heating (cooking) grain with appropriate amount of water.

Besides the above-mentioned steps, a cooling step, a step of adding other liquid seasoning and additive, a step of mixing with liquid seasoning and the like may be included as appropriate.

In the method of the present invention, the step of contacting glucoamylase and α-glucosidase with the grain is not particularly limited as long as glucoamylase and α-glucosidase can be contacted with the grain. For example, the enzymes may be directly contacted with the grain or enzyme water may be prepared from the enzymes and water and contacted with the grain, before and after the above-mentioned steps (b) and (c).

GA and AG may be contacted with the grain simultaneously or with time difference. For example, GA is contacted in the above-mentioned step (b) and AG is contacted in the above-mentioned step (c) or vice versa; GA is contacted in the above-mentioned step (b) and AG is contacted after the above-mentioned step (c) or vice versa; GA is contacted in the above-mentioned step (c) and AG is contacted after the above-mentioned step (c) or vice versa, and the like can be mentioned. Among these, GA and AG are preferably added together to water in step (b) or (c).

In the present invention, the amount of glucoamylase to be contacted (added) is an amount corresponding to an enzyme activity of 10 - 500U per 100 g of the grain (100 g of uncooked rice as starting material in the case of rice food). Within this range, aging of the grain food can be suppressed, water retention can be enhanced, and good texture can be maintained even after progress of time. When the enzyme amount is a trace amount, an enzyme solution with a measurable concentration is prepared, and the solution may be diluted and added.

1U (unit) of glucoamylase is defined to be an activity to generate a reducing power equivalent to 10 mg of glucose in 30 min from soluble starch under the conditions of pH 5.0, 40°C.

In the method of the present invention, the amount of α-glucosidase to be contacted (added) is an amount corresponding to an enzyme activity of 1000 - 100000U, preferably 1000 - 10000U, per 100 g of the grain (100 g of uncooked rice as starting material in the case of rice food). Within this range, aging of the grain food can be suppressed, water retention can be enhanced, and good texture can be maintained even after progress of time. When the enzyme amount is a trace amount, an enzyme solution with a measurable concentration is prepared, and the solution may be diluted and added.

1U (unit) of α-glucosidase is defined to be an enzyme amount to generate 1 µg of glucose in 60 min under the conditions of pH 5.0, 40°C using α-methyl-D-glucoside as a substrate (reference document; Japan Food Additives Association, Voluntary specifications of existing food additives fourth edition "transglucosidase activity measurement method").

In the method of the present invention, the ratio of glucoamylase and α-glucosidase to be contacted is, from the aspect of aging suppressive effect, generally 1.0x10⁻⁷ - 1.0x10³U, preferably 1.0x10⁻⁵ - 10U, more preferably 1.0x10⁻³ - 5.0x10⁻¹U, relative to 1U α-glucosidase.

The time of contact of each enzyme (reaction time) is not particularly limited as long as the enzyme can act on the grain. From the aspect of cooking and production step of the grain food, it is 10 min - 24 hr, preferably 20 min - 2 hr.

The contact temperature (reaction temperature) is not particularly limited as long as the enzyme can maintain activity. From the aspect of reaction efficiency, it is generally 20 - 70°C, preferably 40 - 60°C.

The pH at which each enzyme is contacted is not particularly limited, and it is generally pH 3 - 10.

In the above-mentioned (a) washing step of grain, the grain is generally washed with water, and the washing time and washing temperature can be appropriately selected according to the grain.

In the above-mentioned step (b) for immersing the grain in water, the immersion time varies depending on the grain. In the case of rice, it is generally 0 - 30°C for 0.1 - 24 hr, preferably 4 - 20°C for 1 - 3 hr.

In the above-mentioned step (c) for heating (cooking) the grain with an appropriate amount of water, from the aspect of texture and property after cooking rise, the amount of water to be added is generally 80 - 200 g (water content ratio 80 - 200 wt%), preferably 100 - 170 g (water content ratio 100 - 170 wt%), more preferably 110 - 160 g (water content ratio 110 - 160 wt%), further preferably 120 - 150 g (water content ratio 120 - 150 wt%), per 100 g of the grain.

For the step of heating the grain, a method of heating at normal pressure can be mentioned; however, heating may be performed under pressurization. It is also possible to cook rice using a conventional rice cooker. The heating time is appropriately selected according to the grain, and fire and the like can also be determined by a conventionally-known method.

The present invention also includes a modification method for a grain food including a step of contacting glucoamylase and α-glucosidase with the grain. The grain food modification method is a method for improving the quality such as texture and the like or production suitability of a grain food. The definition, contact amount and preferably range of each component are as described above.

### [Examples]

The present invention is explained further in the Examples. The sensory evaluation in the Examples was conducted using well-trained professional panelists engaged in the food business for more than 5 years unless otherwise specified. In the present specification, unless otherwise specified, % shows wt%.

### <Preparation of cooked rice>

Water was poured into uncooked rice (450 g) and the rice was washed 5 times by changing water. Thereafter, the rice was immersed in a predetermined amount of water for 1 hr. The immersed rice was poured in a basket to drain water, and the rice after draining water was placed in a rice cooker (3.5-go rice cooker, manufactured by Mitsubishi Electric Corporation). Water was added to the water content ratio described in the Table relative to the uncooked rice weight and the rice was immersed for 1 hr. Each enzyme was added in the amount described in the Table, and cooked in the rice cooker. As glucoamylase, glutase AN (13000 U/g, manufactured by HIB Enzymes Inc.) (hereinafter to be indicated as "GA") is preferable, and as α-glucosidase, transglucosidase is preferable and transglucosidase L "Amano" (600000 U/g, manufactured by Amano Enzyme Inc.) (hereinafter to be indicated as "AG") was used.

### <Evaluation of rice>

After cooking, the cooked rice after preservation was evaluated by the method of each test by not less than 3 panelists according to the following criteria. Average of evaluation is shown in each Table.

### (1) Suppression of aging

Elasticity, stickiness and crumbliness of cooked rice were evaluated.
⊙: extremely good
○: good (elastic or sticky and less crumbly)
Δ: average
x: bad (no elasticity or stickiness but crumbly)

### (2) Water retention over time

Dry feeling of surface after heating cooked rice with microwave oven was evaluated.
⊙: extremely good
○: good (small dry feeling on surface)
Δ: average
x: bad (dry feeling on surface)

### (3-1) Formability (easy working)

Cooked rice was formed into a rice ball, and texture (softness) and shape retainability (crushing) of rice grain were evaluated.
○: good (rice grain is appropriately hard and maintains shape)
Δ: average
x: bad (rice grain is too soft and crushed)

### (3-2) Formability (being loosened)

Vinegared rice was prepared and whether clump of the vinegared rice is easily loosened was evaluated 5 hr later.
○: good (easily loosened)
Δ: average
x: bad (difficult to be loosened)

### (4) Production suitability

Sticking to the bottom of rice cooker pot during cooking rice was evaluated.
○: good (almost no sticking to bottom of rice cooker pot)
Δ: average
x: bad (much sticking to bottom of rice cooker pot)

### <Experimental Example 1>

Blended rice of *Koshihikari* and *Yumepirika* was cooked by the method described in <Preparation of cooked rice> at the water content ratio and enzyme described in Table 1. The cooked rice was vacuum cooled (20°C), dispensed by 240 g in a pack, and preserved at 5°C. One day later, a lid was placed on the cooked rice and the cooked rice was heated in a microwave at 1500W for 1 min. At 10 min after completion of the heating, the lid was opened and the aging suppressive effect was evaluated. In addition, at 10 min after completion of the heating, the lid was opened, the cooked rice was left standing for 10 min and water retention over time was evaluated.

The evaluation of each is shown in Table 1, and overall evaluation was made with the water retention over time as the main evaluation index, and the aging suppressive effect as the secondary evaluation index.

**[Table 1]**

| | no addition | (2) cellulase | (3) hemicellulase | (4) xylanase | (5) α-amylase | (6) β-amylase | (7) glucoamylase |
|---|---|---|---|---|---|---|---|
| water content ratio (per 100% uncooked rice) | 130% | 130% | 130% | 130% | 130% | 130% | 130% |
| enzyme addition unit (per 100 g uncooked rice) | | 0.9 | 70 | 23 | 580 | 140 | 61 |
| aging suppression (texture at 10 min after microwaving) | x | Δ | Δ | Δ | Δ | Δ | ○ |
| water retention over time (texture at 20 min after microwaving) | x | x | x | x | Δ | Δ | ○ |
| overall evaluation | x | x | x | x | Δ | Δ | ○ |

From the results of Table 1, it was shown that the cooked rice added with glucoamylase was superior in both aging suppression and water retention over time.

### <Experimental Example 2>

Blended rice of *Koshihikari* and *Yumepirika* was cooked by the method described in <Preparation of cooked rice> at the water content ratio and enzyme described in Table 2. The cooked rice was vacuum cooled (20°C), dispensed by 240 g in a pack, and preserved at chilled 5°C. One day later, a lid was placed on the cooked rice and the cooked rice was heated in a microwave at 1500W for 1 min. At 10 min after completion of the heating, the lid was opened, and the aging suppressive effect was evaluated. In addition, at 10 min after completion of the heating, the lid was opened, the cooked rice was left standing for 10 min and water retention over time was evaluated.

The evaluation of each is shown in Table 2, and overall evaluation was made with the water retention over time as the main evaluation index, and the aging suppressive effect as the secondary evaluation index.

**[Table 2]**

| | (1) no addition | (2) glucoamylase | (3) glucoamylase + α-glucosidase |
|---|---|---|---|
| water content ratio (per 100% uncooked rice) | 130% | 150% | 150% |
| enzyme addition unit (per 100 g uncooked rice) | | glucoamylase: 87 | glucoamylase: 87 |
| | | | α-glucosidase: 9600 |
| aging suppression (texture at 10 min after microwaving) | x | ○ | ○ |
| water retention over time (texture at 20 min after microwaving) | x | ○ | ⊙ |
| overall evaluation | x | ○ | ⊙ |

From the results of Table 2, it was shown that the water retention over time was particularly improved by the combined use of glucoamylase and α-glucosidase.

### <Experimental Example 3>

*Hitomebore* rice was cooked by the method described in <Preparation of cooked rice> at the water content ratio and enzyme described in Table 3. The cooked rice was vacuum cooled (20°C), formed by 80 g in the shape of rice balls (3 rice balls) and wrapped with a food packaging wrap. The formability into rice ball was evaluated according to the above-mentioned criteria. The rice balls were preserved at ordinary temperature (18°C) and the aging suppressive effect was evaluated after preservation for one day.

The evaluation of each is shown in Table 3, and overall evaluation was made with the aging suppressive effect as the main evaluation index, and formability as the secondary evaluation index.

**[Table 3]**

| | (1) no addition | (2) α-glucosidase | (3) glucoamylase | (4) glucoamylase + α-glucosidase |
|---|---|---|---|---|
| water content ratio (per 100% uncooked rice) | 130% | 145% | 145% | 145% |
| enzyme addition unit (per 100 g uncooked rice) | | α-glucosidase: 17100 | glucoamylase: 198 | glucoamylase: 87 |
| | | | | α-glucosidase: 9600 |
| aging suppression (preservation at ordinary temperature) | x | ○ | ○ | ⊙ |
| formability (easy working) | ○ | Δ | ○ | ○ |
| overall evaluation | x | Δ | ○ | ⊙ |

From the results of Table 3, it was shown that aging is suppressed and rice ball with good formability is obtained by the combined use of glucoamylase and α-glucosidase.

### <Experimental Example 4>

*Masshigura* (brand name) rice was cooked by the method described in <Preparation of cooked rice> at the water content ratio and enzyme described in Table 4. Sticking to the bottom of rice cooker pot during cooking rice was evaluated according to the above-mentioned criteria. Vinegar was added (amount of vinegar was about 10% of the cooked rice), they were mixed for 3 min by a cooked rice-vinegar mixer (manufactured by Suzumo Machinery Co., Ltd., MCR-SSC) to admix vinegar. The obtained vinegared rice was dispensed in a plastic case, preserved in a food tray and a refrigerator (5°C). After 5 hr of preservation, the formability (loosening property) of the food tray-preserved product and the aging suppressive effect of the refrigerator-preserved product were evaluated.

The evaluation of each is shown in Table 4, and overall evaluation was made with the aging suppressive effect as the main evaluation index, and formability and production suitability as the secondary evaluation index.

**[Table 4]**

| | (1) no addition | (2) α-amylase | (3) α-amylase + glucoamylase | (4) α-amylase + glucoamylase + α-glucosidase |
|---|---|---|---|---|
| water content ratio (per 100% uncooked rice) | 130% | 140% | 140% | 140% |
| enzyme addition unit (per 100 g uncooked rice) | | α-amylase: 835 | α-amylase: 835 | α-amylase: 835 |
| | | | | glucoamylase: 87 |
| | | | glucoamylase: 87 | |
| | | | | α-glucosidase: 9600 |
| aging suppression (chilled preservation) | x | Δ | ○ | ⊙ |
| formability (loosening property) | x | ○ | ○ | ○ |
| production suitability (Sticking to bottom of rice cooker pot) | ○ | x | Δ | ○ |
| overall evaluation | x | Δ | ○ | ⊙ |

From the results of Table 4, it was shown that aging is suppressed and vinegared rice superior in formability and production suitability is obtained by the combined use of glucoamylase and α-glucosidase.

### [Industrial Applicability]

According to the present invention, cooked rice and rice processed food having improved texture and quality can be provided.

## Claims

1. A grain food modifying agent comprising glucoamylase and α-glucosidase as active ingredients, wherein a content of the glucoamylase is 1.0x10⁻³ - 5.0x10⁻¹U relative to 1U of α-glucosidase.

2. A grain food comprising the modifying agent according to claim 1.

3. The grain food according to claim 2, wherein the grain food is cooked rice.

4. A method for producing a grain food, comprising a step of contacting glucoamylase and α-glucosidase with the grain, wherein 10 - 500U of glucoamylase is contacted per 100 g of the grain and 1000 - 100000U of α-glucosidase is contacted per 100 g of the grain, and the amount of the glucoamylase is 1.0x10⁻³ - 5.0x10⁻¹U per 1U of α-glucosidase.

5. The method according to claim 4, wherein 1000 - 10000U of α-glucosidase is contacted per 100 g of the grain.

6. The method according to claim 4 or 5, comprising a step of adding 80 - 200 g of water per 100 g of the grain and heating them.

7. The method according to any one of claims 4 to 6, wherein the grain is rice.

## Patentansprüche

1. Modifizierungsmittel für Getreidelebensmittel, umfassend Glucoamylase und α-Glucosidase als Wirkstoffe, wobei ein Gehalt der Glucoamylase 1,0×10⁻³ - 5,0×10⁻¹ U bezogen auf 1 U α-Glucosidase beträgt.

2. Getreidelebensmittel, umfassend das Modifizierungsmittel nach Anspruch 1.

3. Getreidelebensmittel nach Anspruch 2, wobei das Getreidelebensmittel gekochter Reis ist.

4. Verfahren zur Herstellung eines Getreidelebensmittels, umfassend einen Schritt des Kontaktierens von Glucoamylase und α-Glucosidase mit dem Getreide, wobei 10 - 500 U Glucoamylase pro 100 g des Getreides kontaktiert werden und 1000 - 100000 U α-Glucosidase pro 100 g des Getreides kontaktiert werden, und die Menge der Glucoamylase 1,0×10⁻³ - 5,0×10⁻¹ U pro 1 U α-Glucosidase beträgt.

5. Verfahren nach Anspruch 4, wobei 1000 - 10000 U α-Glucosidase pro 100 g des Getreides kontaktiert werden.

6. Verfahren nach Anspruch 4 oder 5, umfassend einen Schritt des Hinzufügens von 80 - 200 g Wasser pro 100 g des Getreides und deren Erhitzung.

7. Verfahren nach einem der Ansprüche 4 bis 6, wobei das Getreide Reis ist.

## Revendications

1. Agent modificateur d'aliment en grains comprenant de la glucoamylase et de l'α-glucosidase en tant qu'ingrédients actifs, dans lequel la teneur en la glucoamylase est de 1,0 x 10⁻³ à 5,0 x 10⁻¹ U pour 1 U d'α-glucosidase.

2. Aliment en grains comprenant l'agent modificateur selon la revendication 1.

3. Aliment en grains selon la revendication 2, lequel aliment en grains est le riz cuit.

4. Procédé pour produire un aliment en grains, comprenant une étape de mise en contact de glucoamylase et d'α-glucosidase avec les grains, dans lequel 10 à 500 U de glucoamylase sont mises en contact par 100 g des grains et 1 000 à 100 000 U de l'α-glucosidase sont mises en contact par 100 g des grains, et la quantité de la glucoamylase est de 1,0 x 10⁻³ à 5,0 x 10⁻¹ U pour 1 U d'α-glucosidase.

5. Procédé selon la revendication 4, dans lequel 1 000 à 10 000 U d'α-glucosidase sont mises en contact par 100 g des grains.

6. Procédé selon la revendication 4 ou 5, comprenant une étape d'addition de 80 à 200 g d'eau par 100 g des grains et de chauffage de ceux-ci.

7. Procédé selon l'une quelconque des revendications 4 à 6, dans lequel le grain est le riz.
